# EUROPÄISCHE PATENTSCHRIFT

(11) **EP 1 486 175 B1**
(45) Veröffentlichungstag und Bekanntmachung des Hinweises auf die Patenterteilung: **04.11.2009**
(21) Anmeldenummer: 04011531.3
(22) Anmeldetag: 14.05.2004
(51) Int. Cl.: A61B 17/80, A61B 17/70

(54) **Osteosyntheseplatte oder vergleichbares Implantat nebst Kugelhülse**
Osteosynthetic plate or similar implant with a spherical sleeve
Plaque d'ostéosynthèse ou implant similaire avec une douille sphérique

(30) Priorität: 11.06.2003 DE 10326643
(43) Veröffentlichungstag der Anmeldung: 15.12.2004
(73) Patentinhaber: Mückter, Helmut, Dr. med. Dipl.-Ing., 52076 Aachen (DE); Hildinger, Karl Heinz, 52080 Aachen (DE)
(72) Erfinder: Mückter, Helmut, Dr. med. Dipl.-Ing., 52076 Aachen (DE)
(74) Vertreter: Castell, Klaus

(56) Entgegenhaltungen:
- WO-A-00/24325
- WO-A-98/51226
- FR-A- 2 778 088
- US-A- 5 616 144
- US-A1- 2001 037 112

## Beschreibung

Die Erfindung betrifft eine Osteosyntheseplatte oder ein vergleichbares Implantat nebst einer Kugelhülse für ein solches Implantat.

In der orthopädischen Chirurgie stellt sich regelmäßig die Aufgabe, durch Bruch voneinander getrennte Knochenfragmente zur Stützung und/oder zur Heilung miteinander zu verbinden. Hierfür haben sich Osteosyntheseplatten und vergleichbare orthopädische Implantate bewährt. Diese verfügen über besonders gestaltete Aufnahmebohrungen zur Aufnahme von Knochenschrauben. Die Implantate sind besonders zur Fixierung von instabilen Brüchen großer oder kleiner Röhrenknochen sowie von gedrungenen Knochen wie etwa Wirbel- Handwurzel- oder Fußwurzelknochen bestimmt.

Zur Stabilisierung von Knochenbrüchen sind zahlreiche Implantate der betroffenen Gattung vorgeschlagen worden, welche grob in drei Gruppen unterteilt werden können:

So sind zunächst herkömmliche Osteosyntheseplatten mit sphärischem oder konischem Schraubensitz oder sphärischem Gleitsitz zur dynamischen Kompression bekannt. Hierbei handelt es sich um einfache Platten aus biokompatiblem Metall, in welche Knochenschrauben durch vorgegebene Löcher in den Knochen eingedreht werden. Die Löcher in den Platten sind unterschiedlich geformt und weisen zur Zentrierung der Schraubenköpfe Senkbohrungen in Form sphärischer oder konischer Rundbohrungen oder sphärische Gleitlöcher auf. Beim Anziehen der Schraube im Knochen wird die Platte gegen den Knochen gezogen und gegen diesen verspannt.

Derartige Platten-Schrauben-Verbindungen lassen fast ausschließlich die Übertragung von Zugkräften in Richtung der Schraubenachse sowie von Querkräften zu. Es besteht bei dieser Art von Platten-Schrauben-Verbindungen jedoch keine Winkelstabilität. Elementare Voraussetzung für die Anwendung von herkömmlichen Osteosyntheseplatten ist daher eine ausreichende Eingriffsfestigkeit der Schraubengewinde im Knochen, welche es gestattet, die Platte ausreichend fest gegen den Knochen zu verspannen. Insbesondere bei Osteoporose und im Gelenkbereich von Röhrenknochen gelingt es jedoch häufig nicht, ausreichende Haltekräfte der Schraubengewinde im Knochen zu erzielen. Es kann dann zur Auslockerung der Schrauben, zum Abkippen der Knochenfragmente gegeneinander und zum Implantatversagen kommen.

Weiterhin sind monoaxial stabile Platten-Schrauben-Verbindungen bekannt. Hier werden Knochenschrauben in einem bestimmten konstruktiv vorgegebenen Winkel durch Bohrungen in der Osteosyntheseplatte in den Knochen eingedreht. Die Bohrungen sind derart gestaltet, dass die Schrauben in diesem vorgegebenen Winkel in der Osteosyntheseplatte verspannt werden. Hierdurch ist es möglich, auch gedrungene Knochenfragmente bei gelenknahen Brüchen in einem festen Winkel zur Knochenplatte zu halten, ohne die Platte gegen den Knochen verspannen zu müssen. Vorteilhaft wird so die Knochenhaut geschont. Solche Implantate sind beispielsweise aus der US 5,053,036, der US 6,468,278 B1 oder der DE 43 41 980 A1 bekannt.

Ein Nachteil der monoaxial stabilen Platten-Schrauben-Verbindung besteht jedoch darin, dass wegen des fest vorgegebenen Platten-Schrauben-Winkels die Osteosyntheseplatte sehr genau am Knochen ausgerichtet werden muss. Bereits geringe Fehllagen der Platte können nicht mehr korrigierbare Fehllagen der Schrauben verursachen.

Schließlich sind aus der US 5,053,036, der FR 2 790 198, der US 5,954,722, der US 5,520,690 und der US 5,607,426 verschiedene Ausführungen von polyaxial stabilen Platten-Schrauben-Verbindungen bekannt. Hier werden die Knochenschrauben in einem innerhalb bestimmter Freiheitsgrenzen wählbaren Winkel durch Bohrungen in der Osteosyntheseplatte in den Knochen eingedreht. Da die Schrauben natürlich zunächst keine Winkelstabilität haben, werden sie mittels arretierbarer Spannhülsen, welche zwischen Schraube und Bohrung in der Platte liegen, in dem zuvor eingestellten Winkel verklemmt. Dabei weisen die äußere Oberfläche der Spannhülse und/oder die äußere Oberfläche des Schraubenkopfes eine sphärische Form auf, um eine freie Winkelbeweglichkeit zu gewährleisten.

Einen alternativen Weg zur freien Winkelwahl ohne Notwendigkeit von Spannhülsen zeigen Wolter, Schümann, Seide (1999): The Universal Titanium Fixator; Trauma Berufskrankheiten 1, 307-19. Hier wird mit einem Werkzeug spanfrei ein Gewinde in der Bohrung erzeugt, durch welches anschließend eine Knochenschraube verschraubt wird. Beim Erzeugen des Gewindes wird jedoch die endgültige Schraubenrichtung ohne Spielraum festgelegt.

Bei den benannten Implantaten ist grundsätzlich zu unterscheiden zwischen solchen, welche Zugkräfte auf den Knochen ausüben können, und solchen, welche lediglich in den Knochen eingedreht werden. Bei einer Vielzahl von Indikationen ist es für die Frakturreposition von Bedeutung, das Knochenfragment durch Andrehen der Knochenschraube gegen die Osteosyntheseplatte gezielt zu verspannen, um so eine von der Plattenform vorgegebene anatomiegerechte Position des jeweiligen Knochenfragments zu erreichen. Von den angeführten Ausführungen ist dies nur bei Implantaten nach der US 5,520,690 und der sehr ähnlichen US 5,607,426 möglich. Die dort vorgeschlagenen Implantate weisen jedoch konstruktionsbedingt eine relativ große Bauhöhe im Bereich des Schraubenkopfes auf, wodurch das Einsatzspektrum insbesondere bei ungünstiger Weichteildeckung begrenzt wird.

Die EP 0 201 024 A1 offenbart zum Fixieren von Knochenschrauben in beliebigen Richtungen eine Anordnung, in welcher eine erste Platte am Knochen anliegt und in einer Aufnahmebohrung eine Knochenschraube polyaxial aufnimmt, wobei die Aufnahmebohrung zum Kopf der Knochenschraube so dimensioniert ist, dass der Kopf zur knochenabgewandten Seite über die erste Platte übersteht. Auf diesen überstehenden Kopf soll nach Anziehen der Knochenschraube eine Deckplatte gelegt und diese durch Verschrauben gegen die erste Platte gezogen werden. Infolge der Pressung des Knochenschraubenkopfes durch die Deckplatte wird zwischen dem Knochenschraubenkopf und der ersten Platte ein Reibschluss erzeugt, welcher nach Angabe in der Druckschrift eine richtungssteife Verbindung bewirkt. Auch hier ist jedoch konstruktionsbedingt eine relativ große Bauhöhe nicht zu vermeiden. Zudem ist die Konstruktion relativ aufwendig und somit teuer in der Herstellung.

Die US 5,616,144 sowie die daraus abgeleitete weitgehend inhaltsgleiche EP 0 599 640 B1 (Deutsche Übersetzung DE 693 20 593 T2) offenbaren in Fig. 7a und Fig. 7b eine Verklemmung einer Knochenschraube in einer Osteosyntheseplatte, bei der der Querschnitt der Schraubenaufnahmebohrung durch Verdrehen eines Nocken in einer daneben angeordneten Bohrung durch Verbiegen eines auslenkbaren Armes verkleinert wird. Hierdurch wird der Schraubenkopf in der Schraubenaufnahmebohrung verklemmt, wodurch ein Verdrehen und ein axiales Verschieben der Schraube verhindert werden soll. Nachteilig bei dieser Technik ist, dass die passgenau zur Aufnahmebohrung auszulegende Knochenschraube im Wesentlichen nur senkrecht zur Platte eingedreht werden kann, da sie ansonsten beim Eindrehen am Rand der Aufnahmebohrung in der Knochenplatte auflaufen würden. Ein weiterer Nachteil dieser Gestaltung besteht darin, dass mit einem Nocken in der dargestellten Art keine ausreichende Selbsthemmung erzielbar ist und somit unter dynamischer Belastung rasch eine Lockerung der Verklemmung eintreten würde. Als weiterer Nachteil dieser Gestaltung ist anzusehen, dass sowohl im Bereich des Nockens als auch im Bereich der Aufnahmebohrung die Osteosyntheseplatte zum Seitenrand hin offen gestaltet ist, was zu einer erheblichen Schwächung der Biegefestigkeit der Platte führt.

Die W 00/24325 offenbart in Fig. 23 und Fig. 24 ein Verfahren zur Sicherung einer Knochenschraube in einer Osteosyntheseplatte, wobei nach Positionierung der Knochenschraube im Knochen eine in die Platte integrierte Lasche unter plastischer Verformung über den Schraubenkopf gebogen wird und so ein Herausgleiten des Schraubenkopfes aus der Osteosyntheseplatte verhindert wird. Eine vollständige Verklemmung des Schraubenkopfes mit Blockierung der Rotation wird hierdurch nicht erreicht. Auch ist bei einer derartigen Gestaltung eine Verkippung des Schraubenkopfes in der Aufnahmebohrung möglich.

Die FR-A-2 778 088 und die daraus abgeleitete weitgehend inhaltsgleiche EP 1 075 226 B1 (Deutsche Übersetzung: DE 699 15 464 T2) offenbaren ein Verfahren zur Sicherung einer Knochenschraube in einer Osteosyntheseplatte, wobei nach Positionierung der Knochenschraube im Knochen ein in die Osteosyntheseplatte integrierter Schieber über den Schraubenkopf gelegt wird. Auf diese Weise wird ein Herausgleiten des Schraubenkopfes aus der Osteosyntheseplatte verhindert. Diese Schieberposition wird durch Eindrehen einer weiteren am anderen Ende des Schiebers positionierten Knochenschraube gesichert. Eine vollständige Verklemmung des Schraubenkopfes mit Blockierung der Rotation wird hierdurch nicht erreicht, da keine Vorrichtung vorhanden ist, welche den Schieber fest gegen die Kno chenschraube presst. Der Schieber hat somit lediglich eine Platzhalterfunktion im Sinne eines einfachen Riegels.

Vorliegender Erfindung liegt die Aufgabe zugrunde, ohne Einschränkung der Winkelfreiheit für die Knochenschrauben - beziehungsweise ohne Einschränkung der üblichen Verwendung eines jeweiligen Implantattyps - ein Implantat so auszugestalten, dass die Knochenschrauben bei möglichst geringer Plattendicke und möglichst geringer Gesamtbauhöhe im Bereich der Knochenschrauben besonders zuverlässig und dennoch einfach fixiert werden können. Eine beispielhafte übliche Verwendung eines Implantattyps soll dabei insbesondere auch die Möglichkeit sein, über die Knochenschraube beim Eindrehen in den Knochen Zugkräfte auf diesen ausüben zu können, sodass der Knochen an die Osteosyntheseplatte herangezogen und eine Verspannung zwischen Knochen und Osteosyntheseplatte erreicht werden kann.

Die gestellte Aufgabe löst eine Osteosyntheseplatte oder ein vergleichbares orthopädisches Implantat gemäß Anspruch 1. Vorteilhafte Ausgestaltungen können beispielsweise den Unteransprüchen entnommen werden.

Unter einem indirekten Halten der Knochenschraube wird verstanden, dass zwischen der Knochenschraube und der Aufnahmebohrung eine Klemmhülse positioniert ist, welche ihrerseits in der Aufnahmebohrung liegt, wobei mindestens eines der Bauelemente Aufnahmebohrung oder eingesetzte Klemmhülse sphärisch gestaltet ist.

Als eingeengt wird die Aufnahmebohrung bezeichnet, wenn sich ein freier Durchmesser der Aufnahmebohrung verkleinert. Insbesondere kann die Aufnahmebohrung ihre vormals kreisrunde Form verlieren, sodass auch eine die Aufnahmebohrung ursprünglich in ihrem freien Durchmesser ausfüllende Knochenschraube beziehungsweise Klemmhülse eine entsprechende - mitunter im Wesentlichen radiale - Verformung erfährt, woraufhin ein entsprechend großer Reibschluss die Schraube hält.

Eine Aufweitung der Klemmbohrung sei dann vorhanden, wenn sich zumindest ein Teil der Klemmbohrung gegenüber dem ursprünglichen Zustand radial vergrößert. Rein beispielhaft sei hier an eine Aufweitung gedacht, beispielsweise durch Eindrehen einer Klemmschraube in eine Klemmbohrung, wenn mindestens eines der Bauelemente konisch ausgestaltet ist.

Eine Ausnehmung sei dabei jede Querschnitts- oder Materialänderung in der Platte, welche die Platte gegen Biegung schwächt, in welchem also gegenüber einem ansonsten an der Platte vorhandenen oder insbesondere einem benachbarten Bereich das Flächenträgheitsmoment und/oder der E-Modul reduziert ist. Bei gleichbleibendem Querschnitt kann eine Ausnehmung daher auch durchaus ein mit dem Metall der Platte bündig liegendes Element schwächeren Materials sein. Leichter herzustellen ist natürlich eine direkte flächenträgheitsmomentwirksame Querschnittsreduzierung des Plattenmaterials. Durch eine Ausnehmung, also eine gezielte Schwächung im Plattenkorpus, wird der Einflussbereich der Verformung an der Klemmbohrung gezielt in Richtung der Schwächung gelenkt, sodass eine besonders große Verformung, resultierend also eine besonderes große Einengung, an der Aufnahmebohrung vonstatten geht. Außerdem werden andere Bereiche der Platte möglichst wenig verformt und eine Bruchgefahr der Platte somit bestmöglich vermieden. Die gezielte Lenkung der Verformung ist besonders dann zu beobachten, wenn die Ausnehmung zwischen den Bohrungen liegt. Aber bereits eine Ausnehmung im bloßen Bereich der Klemmbohrung kann eine stärkere Verformung der gewünschten Art bewirken.

Die Klemmkräfte, welche durch die Erfindung an der Aufnahmebohrung erzielt werden, sind bei geeigneter Gestaltung um Dimensionen größer als nach dem Stand der Technik bekannte Haltekräfte. Insofern kann der Verwender des erfindungsgemäßen Implantats die unabhängige Verspannmöglichkeit der Knochenschraube in der Aufnahmebohrung gut dazu nutzen, mit hervorragender Feinabstimmung die Platte und/oder die Knochenschrauben zunächst auszurichten und gegebenenfalls durch Anziehen der Knochenschrauben den Knochen gegen das Implantat zu verspannen. Beim endgültigen Verklemmen der Knochenschrauben muss der Verwender dann auf die Knochenschrauben keine direkten Kräfte mehr ausüben, welche die Knochenschrauben etwa in ihrer Lage gegenüber dem Knochen oder der Platte verschieben könnten. Vielmehr ermöglicht es die Erfindung dem Verwender, auch sehr große Klemmkräfte einfach indirekt aufzubringen. Zudem ist die erfindungsgemäße Verklemmmöglichkeit unabhängig von der jeweiligen Ausrichtung der Knochenschrauben. Dies ist besonders bei polyaxial fixierbaren Schrauben von großem Vorteil.

Dabei kann der Verwender ein in eine Klemmbohrung eingesetztes Klemmelement besonders gut erreichen, wenn dieses an der Oberseite der Platte zugänglich ist. Dies wird dadurch erreicht, dass die Klemmbohrung im Wesentlichen senkrecht zur knochenabgewandten Oberfläche der Platte verläuft. Sofern eine Aufnahmebohrung nicht im Wesentlichen senkrecht, sondern unter einem schrägen Winkel relativ zur Plattenoberfläche angeordnet ist, kann es vorteilhaft sein, wenn die Klemmbohrung mit ihrer Achse zumindest im Wesentlichen parallel zur Achse der Aufnahmebohrung verläuft.

Um die Klemmkraft genauer justieren zu können und verklemmte Schrauben problemlos wieder lösen zu können, wird zudem vorgeschlagen, dass bei elastischer Aufweitung der Klemmbohrung auch die Einengung der Aufnahmebohrung elastisch ist. Insbesondere soll also keine plastische Verformung im Einflussbereich der aufgeweiteten Klemmbohrung stattfinden.

Unabhängig hiervon ist bei einer bevorzugten Ausführungsform die Klemmbohrung neben der zugehörigen Aufnahmebohrung derart angeordnet, dass der Abstand zwischen den äußeren Durchmessern von Klemmbohrung und Aufnahmebohrung an seiner schmalsten Stelle höchstens einen Klemmbohrungsdurchmesser, bevorzugt höchstens einen halben Klemmbohrungsdurchmesser, besonders bevorzugt höchstens einen viertel Klemmbohrungsdurchmesser, auch bevorzugt höchstens 2 mm, beträgt. Hierdurch wird sichergestellt, dass die Aufnahmebohrung im Einflussbereich der von der Klemmbohrung ausgehenden Verformung liegt, bevor sich bei weiterem Abstand zur Klemmbohrung die Verformung zu sehr in die Fläche der Platte verteilt hat und die lokalen Verformungen nur noch sehr kleine Dimensionen annehmen.

Damit die zur effektiven Einklemmung einer Knochenschraube und einer ggf. vorgesehenen Spannhülse erforderlichen Verformungen der Aufnahmebohrung und der Klemmbohrung mit geringem Kraftaufwand erreicht werden, wird insbesondere vorgeschlagen, dass sowohl die Aufnahmebohrung als auch die Klemmbohrung mit einer Ausnehmung in der Platte unmittelbar in Verbindung stehen. Eine besonders effektive Ausnehmung stellt dabei ein den Plattenquerschnitt durchdringender Schlitz dar.

Eine bevorzugte Ausführungsform sieht vor, dass eine Ausnehmung derart angeordnet ist, dass sie die Aufnahmebohrung und die Klemmbohrung miteinander verbindet. Dabei kann die Ausnehmung symmetrisch zur Verbindungsachse zwischen den Mittelpunkten von Aufnahme- und Klemmbohrung angeordnet sein. Effektiver ist jedoch eine Ausnehmung, welche sowohl die Aufnahmebohrung als auch die Klemmbohrung im Bereich ihrer

Außendurchmesser im Wesentlichen tangential durchdringt. Hierbei verbleibt zwischen Aufnahmebohrung und Klemmbohrung ein schmaler Steg, welcher einseitig eine Basis aufweist. Ein solcher Steg lässt sich mit geringem Kraftaufwand von der Klemmbohrung zur Aufnahmebohrung hin biegen, wodurch die zur Einspannung der Knochenschraube und einer ggf. angeordneten Klemmhülse erforderliche Einengung der Aufnahmebohrung erreicht wird.

Der beschriebene Effekt wird durch eine Zusatzausnehmung im Bereich der Basis des zwischen Aufnahmebohrung und Klemmbohrung gelegenen Steges noch verstärkt. Ein derartige Zusatzausnehmung verringert das Flächenträgheitsmoment an der Basis des Steges. Der zwischen der Klemmbohrung und der Aufnahmebohrung gelegene Steg kann dadurch leichter um die verbliebene Basis gebogen werden.

Besonders effektiv lässt sich eine Einengung der Aufnahmebohrung auch dadurch bewerkstelligen, dass zwischen Klemmbohrung und Aufnahmebohrung ein beweglicher Klemmkörper, etwa ein Schlitten oder eine bewegliche Spannbacke, vorgesehen ist. Dabei ist es vorteilhaft, wenn der bewegliche Spannkörper Bestandteil beider Bohrungswandungen ist. Die Hauptklemmkraft fließt dann über den beweglichen Spannkörper zur Aufnahmebohrung, sobald der Spannkörper von der Achse der Klemmbohrung weiter entfernt wird. Vorzugsweise wird der bewegliche Spannkörper dabei in einer Ausnehmung geführt, wobei diese Ausnehmung beide Bohrungen verbinden kann.

Die Erfindung wird nachstehend anhand der Zeichnung weiter erläutert. Hierin zeigen zu mehreren Ausführungsbeispielen
- Figur 1: in einer teilgeschnittenen Seitenansicht einen Ausschnitt einer ersten Osteosyntheseplatte mit zwei sphärischen Aufnahmebohrungen, zwei jeweils benachbarten Klemmbohrungen, einer verklemmten Knochenschraube, einer separaten Klemmhülse und einer separaten Klemmschraube,
- Figur 2: den Ausschnitt der ersten Osteosyntheseplatte aus Figur 1 in einer Draufsicht,
- Figur 3: in einer Draufsicht einen Ausschnitt einer zweiten Osteosyntheseplatte mit einer Klemmbohrung und zwei über Ausnehmungsschlitze unmittelbar verbundene Aufnahmebohrungen,
- Figur 4: in einer Draufsicht einen Ausschnitt einer dritten Osteosyntheseplatte mit einer Klemmbohrung, welche über einen Ausnehmungsschlitz mit einer Aufnahmebohrung verbunden ist und zugleich eine Zusatzausnehmung aufweist,
- Figur 5: in einer Draufsicht eine Abwicklung einer vierten, H-förmigen Osteosyntheseplatte mit zwei verschiedenen KlemmbohrungsAufnahmebohrungs-Anordnungen,
- Figur 6: in einer Draufsicht einen Ausschnitt einer fünften Osteosyntheseplatte mit einem in der Platte geführten schlittenförmigen Klemmkörper zwischen einer Klemmbohrung und einer Aufnahmebohrung,
- Figur 7: in einer teilgeschnittenen Seitenansicht einen Ausschnitt einer sechsten Osteosyntheseplatte, die nicht unter die Erfindung fällt, mit einer verklemmten Knochenschraube in einer Klemmhülse in einer Aufnahmebohrung und eine Klemmschraube in einer senkrecht zur Aufnahmebohrung und parallel zur Plattenoberfläche verlaufenden Klemmbohrung,
- Figur 8: eine schematische Draufsicht auf die Platte aus dem Ausschnitt aus Figur 7 und
- Figur 9: eine Abwicklung einer siebten, T-förmigen Osteosyntheseplatte mit drei gleichartigen Anordnungen aus Klemmbohrung, Aufnahmebohrung und Ausnehmungsschlitz.

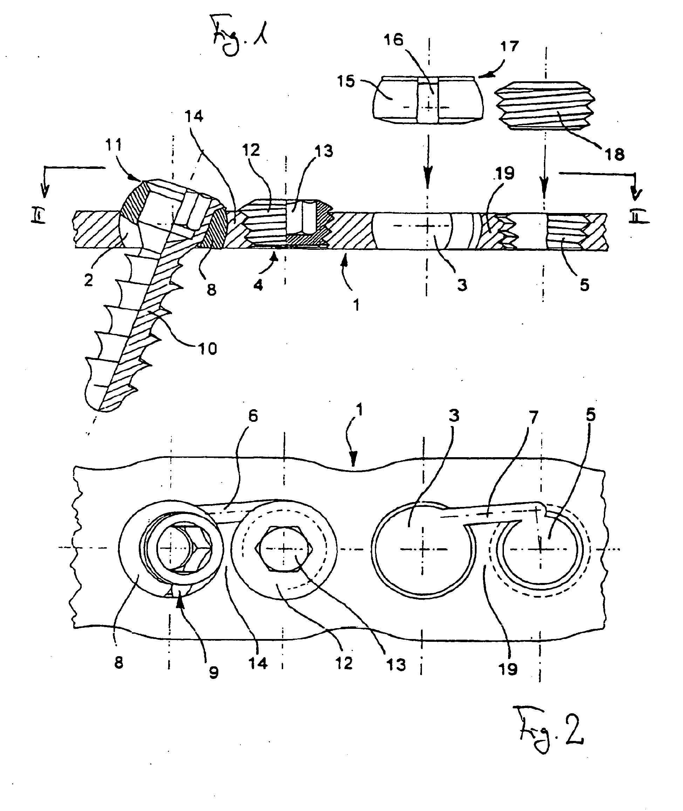

Die Osteosyntheseplatte 1 in den Figuren 1 und 2 weist zwei sphärische Aufnahmebohrungen 2, 3 und zwei Klemmbohrungen 4, 5 in Gestalt von Kegelgewindebohrungen mit zu den Aufnahmebohrungen 2, 3 parallel verlaufenden Bohrungsachsen auf. Jede der beiden sphärischen Aufnahmebohrungen 2, 3 ist durch eine schlitzförmige Ausnehmung 6, 7 mit jeweils einer der Klemmbohrungen 4, 5 verbunden. Dabei liegen die schlitzförmigen Ausnehmungen 6, 7 zwischen den jeweiligen Bohrungen.

In der sphärischen Aufnahmebohrung 2 ist die Kugelhülse 8 passgenau drehbar und in alle Richtungen schwenkbar eingesetzt. Die Kugelhülse 8 weist einen Schlitz 9 auf, welcher eine geringe Verformung der Kugelhülse 8 ermöglicht. Die Kugelhülse 8 weist des weiteren zentral eine zylindrische Bohrung auf, in die passgenau, im Klemm- oder Spielsitz drehbar und verschiebbar die halb geschnittene Knochenschraube 10 eingeführt ist.

An ihrem oberen Ende weist die Kugelhülse 8 einen Kreisringkragen 11 auf, welcher die ansonsten frei wählbare Schwenkbewegung der Schraubenachse auf das konstruktiv gewünschte Maß begrenzt.

In die Klemmbohrung 4 ist als Klemmschraube ein kurzer Kegelgewindebolzen 12 mit Innensechskant 13 eingedreht. Durch Eindrehen des Kegelgewindebolzens 12 in die Klemmbohrung 4 wird ein Steg 14 zur sphärischen Aufnahmebohrung hin gedrückt, wodurch die Osteosyntheseplatte 1, die Kugelhülse 8 und die Knochenschraube 10 gegeneinander verspannt werden. Auf diese Weise resultiert bei festem Andrehen des Kegelgewindebolzens 12 eine winkel-, axial- und rotationsstabile Einspannung der Knochenschraube 10 bezüglich der Platte 1 in der zuvor eingestellten Schwenkstellung der Knochenschraube 10.

Zur Übertragung besonders hoher Einspannmomente ist es vorteilhaft, wenn die Kontaktflächen zwischen sphärischer Bohrung 2 und Kugelhülse 8 eine raue Oberflächenstruktur aufweisen, wodurch der Reibungskoeffizient erhöht wird.

Die sphärische Aufnahmebohrung 3 ist leer. Sie dient zur Aufnahme der noch separaten Kugelhülse 15, welche baugleich mit der Kugelhülse 8 ist. Die Kugelhülse 15 wird entlang der Pfeilrichtung in die Aufnahmebohrung 3 eingeführt. Hierzu muss sie unter Verengung eines Schlitzes 16 so weit zusammengedrückt werden, dass ihr größter Durchmesser den oberen Rand der sphärischen Aufnahmebohrung passieren kann. Nach Einführen der Kugelhülse 15 wird diese wieder entspannt und federt entsprechend auseinander, wodurch sie exakt in der Aufnahmebohrung 3 allein begrenzt durch einen Kreisringkragen 17 schwenkbar geführt ist.

Die Klemmbohrung 5 ist ebenfalls leer. Der noch separate Kegelgewindebolzen 18 (baugleich mit dem Bolzen 12) wird in Pfeilrichtung in die Klemmbohrung 5 eingeschraubt, wodurch ein Steg 19 zur sphärischen Aufnahmebohrung 3 hin gebogen wird.

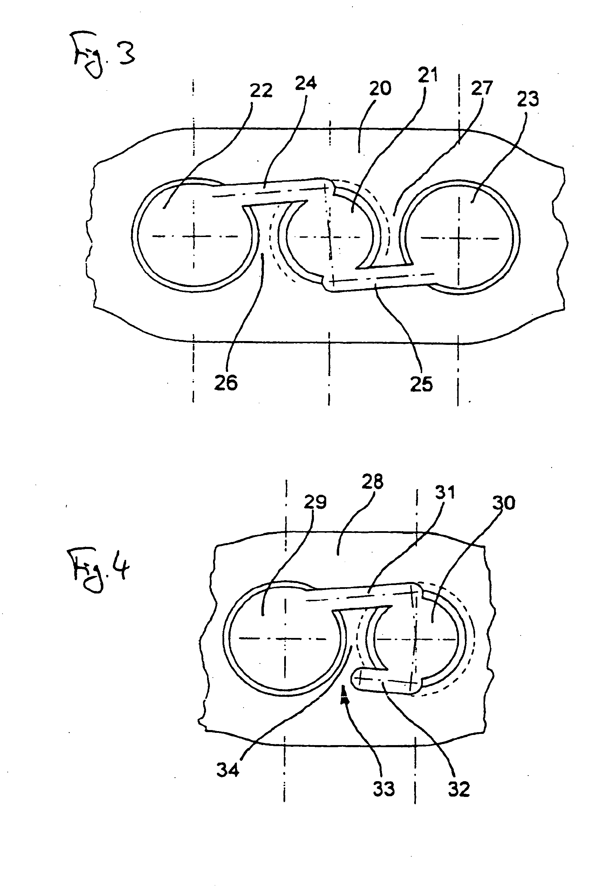

In der zweiten Osteosyntheseplatte 20 in Figur 3 sind zentral die als Kegelgewindebohrung ausgeführte Klemmbohrung 21 und symmetrisch dazu die zwei Aufnahmebohrungen 22, 23 angeordnet. Beide Aufnahmebohrungen 22, 23 sind über je eine schlitzförmige Ausnehmung 24, 25 mit der Klemmbohrung 21 unmittelbar verbunden. Dabei sind die schlitzförmigen Ausnehmungen 24, 25 länglich und jeweils gerade und tangential an die Klemmbohrung 21 angeschlossen. Beim Eindrehen eines Kegelgewindebolzens in die Klemmbohrung 21 werden zwei Stege 26, 27 jeweils zu den Aufnahmebohrungen 22, 23 hin gedrückt.

Auf diese Weise lassen sich jeweils zwei Knochenschrauben durch eine Klemmschraube verklemmen. Voraussetzung ist allerdings, dass beide Aufnahmebohrungen gefüllt sind. Hierzu kann jedoch ein Füllelement vorgesehen sein, welches ohne weitere Funktion lediglich eine Aufnahmebohrung füllt.

Bei der dritten Platte 28 in Figur 4 ist die Aufnahmebohrung 29 mit der Klemmbohrung 30 über die schlitzförmige Ausnehmung 31 unmittelbar, im Wesentlichen tangential, verbunden. Zusätzlich ist zwischen den Bohrungen noch die Zusatzausnehmung 32 angeordnet, welche nur an die Klemmbohrung 30 angeschlossen ist, aber den Plattenquerschnitt an einer Basis 33 eines Steges 34 der Platte 28 so weit schwächt, dass sich der Steg 34 aufgrund des reduzierten Flächenträgheitsmomentes mit geringer Kraft zur Aufnahmebohrung 29 hin biegen lässt.

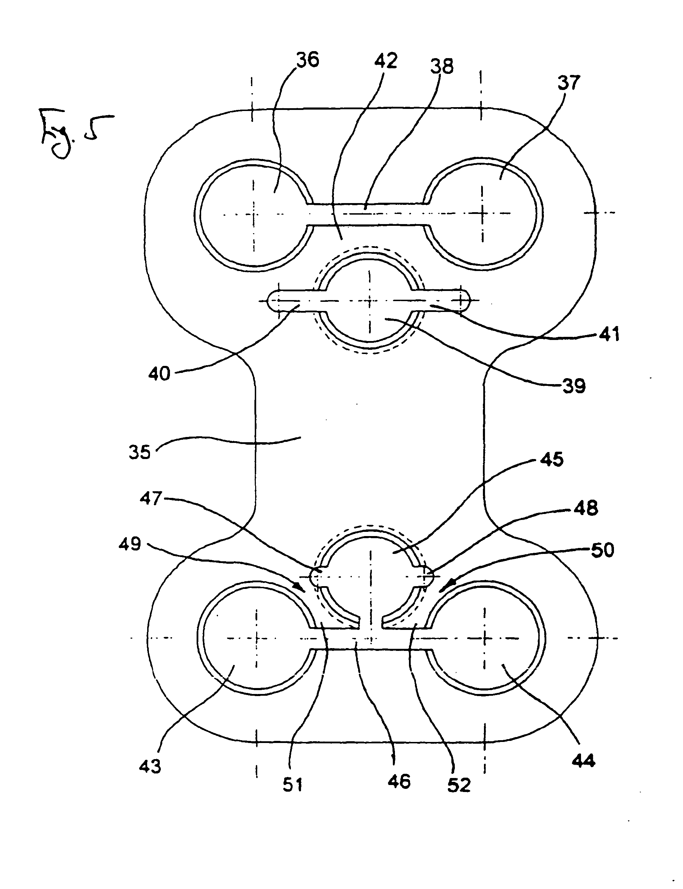

Die vierte Platte 35 in Figur 5 dient bevorzugt zur ventralen Stabilisierung im Bereich der Halswirbelsäule. Im oberen und im unteren Teil der H-Platte 35 sind beispielhaft zwei verschiedenartige Varianten polyaxial winkelstabiler Schraubenaufnahmen dargestellt. Im oberen Abschnitt sind zwei sphärische Aufnahmebohrungen 36, 37 angeordnet, welche durch eine gerade schlitzförmige Ausnehmung 38 miteinander verbunden sind. Symmetrisch hierzu ist eine Klemmbohrung 39 angeordnet. Die Klemmbohrung 39 weist zwei schlitzförmige Ausnehmunden 40, 41 auf, die parallel zu der schlitzförmigen Ausnehmung 38 angeordnet sind. Wie vorstehend beschrieben werden in die Aufnahmebohrungen 36, 37 Kugelhülsen eingesetzt. Durch festes Eindrehen einer kurzen kegelförmigen Klemmschraube in die Klemmbohrung 39 wird ein Steg 42 zum oberen Rand der H-Platte 35 hin gebogen, wodurch die schlitzförmige Ausnehmung 38 und die beiden Aufnahmebohrungen 36, 37 verengt werden. Hierdurch werden eingesetzte Kugelhülsen und Knochenschrauben sicher in der Platte 35 verklemmt.

Im unteren Abschnitt der Platte 35 sind zwei Aufnahmebohrungen 43, 44 sowie symmetrisch hierzu eine Klemmbohrung 45 vorhanden. Die beiden Aufnahmebohrungen 43, 44 und die Klemmbohrung 45 sind durch eine T-förmige Ausnehmung 46 miteinander verbunden. Die Klemmbohrung weist darüber hinaus zwei kurze Zusatzausnehmungen 47, 48 auf, welche jeweils Basen 49, 50 von Stegen 51, 52 schmälern und so das Flächenträgheitsmoment reduzieren.

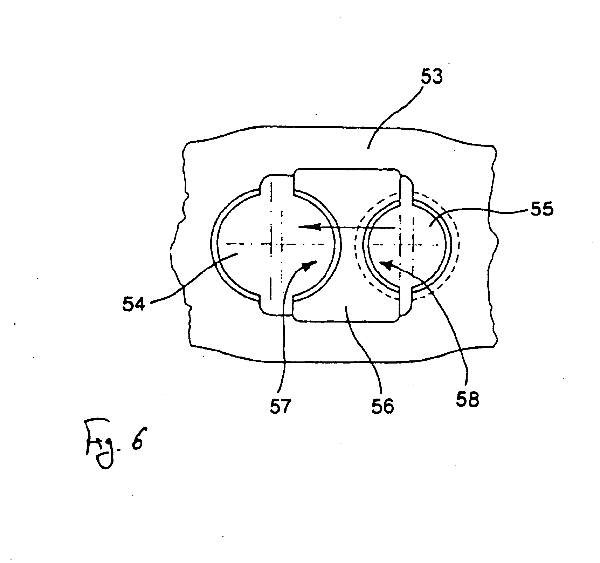

Die fünfte Platte 53 in Figur 6 weist abermals eine sphärische Aufnahmebohrung 54 und eine Kegelgewindebohrung 55 auf, welche aber jeweils nur etwa zur Hälfte ausgebildet sind. Zwischen der jeweils etwa zur Hälfte ausgebildeten Aufnahmebohrung 54 und der Klemmbohrung 55 ist eine Gleitführung für einen beweglichen schlittenförmigen Klemmkörper 56 vorgesehen, welcher in der Platte 53 in Pfeilrichtung hinwärts und rückwärts verschiebbar geführt ist.

Der schlittenförmige Klemmkörper weist an einem Ende eine etwa zur Hälfte ausgebildete sphärische Aufnahmebohrung 57 auf, welche kongruent zu der Aufnahmebohrung 54 gestaltet ist und an dem anderen Ende ein etwa zur Hälfte ausgebildetes konisches Klemmgewinde 58 auf, welches kongruent zu den Gewindegängen des Kegelgewindes, 55 gestaltet ist. Durch festes Eindrehen eines Gewindebolzens oder eines Kegelgewindebolzens wird der Durchmesser der Klemmbohrung 55, 58 vergrößert und der schlittenförmige Klemmkörper 56 in Pfeilrichtung gegen eine in die Aufnahmebohrung 54 eingesetzte Kugelhülse (nicht dargestellt) geschoben, woraus wiederum eine feste Einklemmung der Schraube in die Platte 53 resultiert.

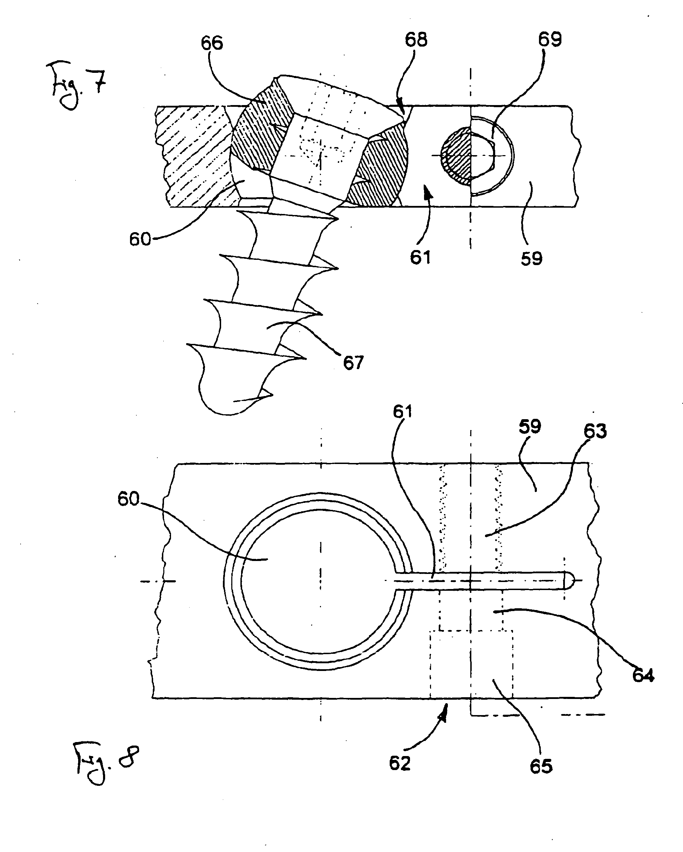

Die sechste Platte 59 in den Figuren 7 und 8 weist wiederum eine sphärische Aufnahmebohrung 60 auf. Zu dieser ist seitlich eine schlitzförmige Ausnehmung 61 angeordnet. Quer zu der schlitzförmigen Ausnehmung 61 ist eine Schraubenaufnahme 62 vorhanden, welche auf der einen Seite der schlitzförmigen Ausnehmung 61 ein Gewinde 63 und auf der andere Seite ein Gleitloch 64 und eine Schraubenkopfaufnahme 65 aufweist.

In eine Kugelhülse 66 ist eine Knochenschraube 67 eingesetzt (nicht dargestellt in Figur 8), wobei die hieraus entstandene Einheit bis zur Begrenzung durch den Kreisringkragen 68 an der Kugelhülse 66 frei schwenkbar ist.

In die in der Platte 59 verlaufende Schraubenaufnahme 62 ist eine Klemmschraube 69 eingesetzt (nicht dargestellt in Figur 8), welche als Innensechskantschraube mit zylindrischem Kopf ausgeführt ist. Durch Andrehen der Klemmschraube 69 werden die schlitzförmige Ausnehmung 61 und folglich die Aufnahmebohrung 60 verengt, wodurch eine Verklemmung zwischen der Schraube 67, der geschlitzten Hülse 66 und der Platte 59 sichergestellt wird.

Die Ausführungsform aus den Figuren 7 und 8 fällt nicht unter die hier zu schützende Erfindung.

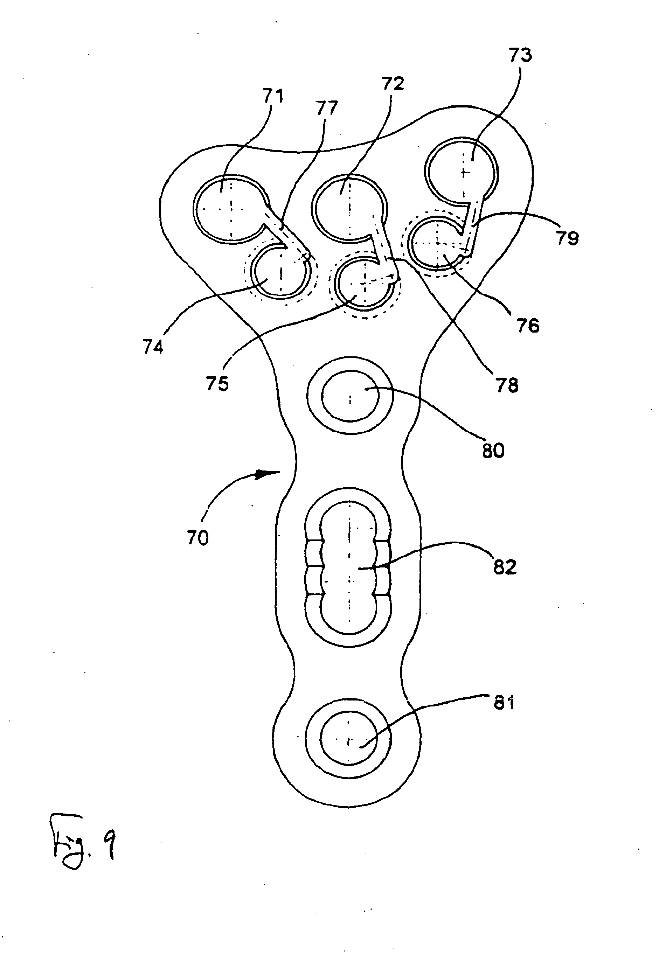

Die siebte Platte 70 in Figur 9 findet bevorzugt zur Stabilisierung von Knochenbrüchen im Bereich des distalen Radius Verwendung. Dabei sind im verbreiterten Abschnitt der Platte 70 drei sphärische Aufnahmebohrungen 71, 72, 73 und drei zugehörige als Kegelgewindebohrungen ausgestaltete Klemmbohrungen 74, 75, 76 angeordnet, welche jeweils durch schlitzförmige Ausnehmungen 77, 78, 79 miteinander verbunden sind. Die Klemmfunktion ist bereits eingehend erläutert.

Im schlanken Teil der Platte 70 sind zusätzlich zwei herkömmliche Rundlöcher 80, 81 mit kugelförmigen Senkbohrungen zur Aufnahme herkömmlicher Schraubenköpfe und ein gleichermaßen gestaltetes Langloch 82 angeordnet.

## Patentansprüche

1. Osteosyntheseplatte (1, 20, 28, 35, 53, 70) oder vergleichbares orthopädisches Implantat mit einer Aufnahmebohrung (2, 3, 22, 23, 29, 36, 37, 43, 44, 54, 71, 72, 73) und einer daneben angeordneten Klemmbohrung (4, 5, 21, 30, 39, 45, 55, 74, 75, 76) und mit einer Ausnehmung (6, 7, 24, 25, 31, 40, 41, 46, 47, 48, 77, 78, 79) im Bereich der Bohrungen, wobei die Klemmbohrung(4, 5, 21, 30, 39, 45, 55, 74, 75, 76) im Wesentlichen senkrecht zur Osteosyntheseplatte (1, 20, 28, 35, 53, 70) oder parallel zur Aufnahmebohrung (2, 3, 22, 23, 29, 36, 37, 43, 44, 54, 71, 72, 73) verläuft und eine Aufweitung der Klemmbohrung (4, 5, 21, 30, 39, 45, 55, 74, 75, 76) eine Einengung der Aufnahmebohrung (2, 3, 22, 23, 29, 36, 37, 43, 44, 54, 71, 72, 73) bewirkt, ***dadurch gekennzeichnet, dass***
- die Klemmbohrung (4, 5, 21, 30, 39, 45, 55, 74, 75, 76) ein konisches Innengewinde zur Aufnahme einer konischen Klemmschraube aufweist, sodass beim Eindrehen der konischen Klemmschraube die Klemmbohrung (4, 5, 21, 30, 39, 45, 55, 74, 75, 76) zur Aufnahmebohrung (2, 3, 22, 23, 29, 36, 37, 43, 44, 54, 71, 72, 73) hin aufgeweitet wird, sowie dass
- in der Aufnahmebohrung (2, 3, 22, 23, 29, 36, 37, 43, 44, 54, 71, 72, 73) eine Kugelhülse (8) zur Aufnahme einer Knochenschraube (10) eingesetzt ist.

2. Osteosyntheseplatte oder vergleichbares orthopädisches Implantat nach Anspruch 1, ***dadurch gekennzeichnet, dass*** die Aufnahmebohrung (2, 3, 22, 23, 29, 36, 37, 43, 44, 54, 71, 72, 73) und die Kugelhülse (8) sphärisch gestaltet sind.

3. Osteosyntheseplatte oder vergleichbares orthopädisches Implantat nach Anspruch 1 oder 2, ***dadurch gekennzeichnet, dass*** die in der Aufnahmebohrung (2, 3, 22, 23, 29, 36, 37, 43, 44, 54, 71, 72, 73) eingesetzte Kugelhülse (8) einen Schlitz zur besseren Verformbarkeit aufweist.

4. Osteosyntheseplatte oder vergleichbares orthopädisches Implantat nach Anspruch 1, ***dadurch gekennzeichnet, dass*** innerhalb der Osteosyntheseplatte (1, 20, 28, 35, 53, 70) im Bereich der Aufnahmebohrung (2, 3, 22, 23, 29, 36, 37, 43, 44, 54, 71, 72, 73) und der Klemmbohrung (4, 5, 21, 30, 39, 45, 55, 74, 75, 76) eine oder mehrere Ausnehmungen (6, 7, 24, 25, 31, 40, 41, 46, 47, 48, 77, 78, 79) angeordnet sind, sodass das Flächenträgheitsmoment des zwischen Aufnahmebohrung (2, 3, 22, 23, 29, 36, 37, 43, 44, 54, 71, 72, 73) und Klemmbohrung (4, 5, 21, 30, 39, 45, 55, 74, 75, 76) gelegenen Teils der Osteosyntheseplatte (1, 20, 28, 35, 53, 70) vermindert wird und hierdurch eine zur Aufnahmebohrung (2, 3, 22, 23, 29, 36, 37, 43, 44, 54, 71, 72, 73) hin gerichtete Aufweitung der Klemmbohrung (4, 5, 21, 30, 39, 45, 55, 74, 75, 76) mit geringem Kraftaufwand ermöglicht wird.

5. Osteosyntheseplatte oder vergleichbares orthopädisches Implantat nach Anspruch 1 oder 4, ***dadurch gekennzeichnet, dass*** eine in der Osteosyntheseplatte (1, 20, 28, 35, 53, 70) angeordnete Ausnehmung (6, 7, 24, 25, 31, 40, 41, 46, 47, 48, 77, 78, 79) die Aufnahmebohrung (2, 3, 22, 23, 29, 36, 37, 43, 44, 54, 71, 72, 73) und die zugehörige Klemmbohrung (4, 5, 21, 30, 39, 45, 55, 74, 75, 76) miteinander verbindet.

6. Osteosyntheseplatte oder vergleichbares orthopädisches Implantat nach Anspruch 1, ***dadurch gekennzeichnet, dass*** in der Osteosyntheseplatte (53) zwischen einer Aufnahmebohrung (54) und der zugehörigen Klemmbohrung (55) eine Ausnehmung derart gestaltet ist, dass die Aufnahmebohrung (54) und die zugehörige Klemmbohrung (55) miteinander verbunden sind und zwischen den Bohrungen ein verschiebbarer Klemmkörper (56) angeordnet ist, wobei der Klemmkörper (56) Bestandteil beider Bohrungswandungen ist.

7. Osteosyntheseplatte oder vergleichbares orthopädisches Implantat nach Anspruch 6, **dadurch gekennzeichnet, dass** der Klemmkörper (56) verschiebbar geführt ist.

## Claims

1. An osteosynthesis plate (1, 20, 28, 35, 53, 70) or a comparable orthopaedic implant with a reception bore (2, 3, 22, 23, 29, 36, 37, 43, 44, 54, 71, 72, 73) and with a clamping bore (4, 5, 21, 30, 39, 45, 55, 74, 75, 76) disposed next to it and with a recess (6, 7, 24, 25, 31, 40, 41, 46, 47, 48, 77, 78, 79) in the region of said bores, said clamping bore (4, 5, 21, 30, 39, 45, 55, 74, 75, 76) extending substantially at right angles with respect to said osteosynthesis plate (1, 20, 28, 35, 53, 70) or parallel to said reception bore (2, 3, 22, 23, 29, 36, 37, 43, 44, 54, 71, 72, 73) and a widening of said clamping bore (4, 5, 21, 30, 39, 45, 55, 74, 75, 76) causing a constriction of said reception bore (2, 3, 22, 23, 29, 36, 37, 43, 44, 54, 71, 72, 73),
***characterized in that***
- said clamping bore (4, 5, 21, 30, 39, 45, 55, 74, 75, 76) comprises a conical internal thread for receiving a conical clamping screw so that, when the conical clamping screw is screwed in, the clamping bore (4, 5, 21, 30, 39, 45, 55, 74, 75, 76) is widened in the direction of the reception bore (2, 3, 22, 23, 29, 36, 37, 43, 44, 54, 71, 72, 73), and that
- a ball bushing (8) for receiving a bone screw (10) is inserted in said reception bore (2, 3, 22, 23, 29, 36, 37, 43, 44, 54, 71, 72, 73).

2. The osteosynthesis plate or a comparable orthopaedic implant as set forth in claim 1, ***characterized in that*** the reception bore (2, 3, 22, 23, 29, 36, 37, 43, 44, 54, 71, 72, 73) and the ball bushing (8) are configured to be spherical.

3. The osteosynthesis plate or a comparable orthopaedic implant as set forth in claim 1 or 2, ***characterized in that*** the ball bushing (8) inserted into the reception bore (2, 3, 22, 23, 29, 36, 37, 43, 44, 54, 71, 72, 73) comprises a slot for improved deformability.

4. The osteosynthesis plate or a comparable orthopaedic implant as set forth in claim 1,
***characterized in that*** one or several recesses (6, 7, 24, 25, 31, 40, 41, 46, 47, 48, 77, 78, 79) are arranged inside said osteosynthesis plate (1, 20, 28, 35, 53, 70) in the region of the reception bore (2, 3, 22, 23, 29, 36, 37, 43, 44, 54, 71, 72, 73) and of the clamping bore (4, 5, 21, 30, 39, 45, 55, 74, 75, 76) so that the geometrical moment of inertia of the part of said osteosynthesis plate (1, 20, 28, 35, 53, 70) which lies between the reception bore (2, 3, 22, 23, 29, 36, 37, 43, 44, 54, 71, 72, 73) and the clamping bore (4, 5, 21, 30, 39, 45, 55, 74, 75, 76) is minimized and that a widening of the clamping bore (4, 5, 21, 30, 39, 45, 55, 74, 75, 76) oriented toward said reception bore (2, 3, 22, 23, 29, 36, 37, 43, 44, 54, 71, 72, 73) is made possible with little force application.

5. The osteosynthesis plate or a comparable orthopaedic implant as set forth in claim 1 or 4, ***characterized in that*** a recess (6, 7, 24, 25, 31, 40, 41, 46, 47, 48, 77, 78, 79) disposed in said osteosynthesis plate (1, 20, 28, 35, 53, 70) connects together the reception bore (2, 3, 22, 23, 29, 36, 37, 43, 44, 54, 71, 72, 73) and the associated clamping bore (4, 5, 21, 30, 39, 45, 55, 74, 75, 76).

6. The osteosynthesis plate or a comparable orthopaedic implant as set forth in claim 1,
***characterized in that*** a recess is formed in such a manner in the osteosynthesis plate (53) between a reception bore (54) and the associated clamping bore (55) that said reception bore (54) and the associated clamping bore (55) are connected together and that a slidable clamping body (56) is interposed between said bores, said clamping body (56) being a component part of the two bore walls.

7. The osteosynthesis plate or a comparable orthopaedic implant as set forth in claim 6,
***characterized in that*** the clamping body (56) is guided for slidable movement.

## Revendications

1. Plaque d'ostéosynthèse (1, 20, 28, 35, 53, 70) ou implant orthopédique comparable, avec un trou de logement (2, 3, 22, 23, 29, 36, 37, 43, 44, 54, 71, 72, 73) et avec un trou de serrage (4, 5, 21, 30, 39, 45, 55, 74, 75, 76) disposé à côté du trou de logement et avec un évidement (6, 7, 24, 25, 31, 40, 41, 46, 47, 48, 77, 78, 79) à la hauteur des trous, le trou de serrage (4, 5, 21, 30, 39, 45, 55, 74, 75, 76) s'étendant sensiblement perpendiculairement à la plaque d'ostéosynthèse (1, 20, 28, 35, 53, 70) ou parallèlement au trou de logement (2, 3, 22, 23, 29, 36, 37, 43, 44, 54, 71, 72, 73) et un élargissement du trou de serrage (4, 5, 21, 30, 39, 45, 55, 74, 75, 76) provoquant un rétrécissement du trou de logement (2, 3, 22, 23, 29, 36, 37, 43, 44, 54, 71, 72, 73),
***caractérisée en ce que***
- le trou de serrage (4, 5, 21, 30, 39, 45, 55, 74, 75, 76) comporte un filetage intérieur conique destiné à recevoir une vis de serrage conique de sorte que, lors du vissage de la vis de serrage conique, le trou de serrage (4, 5, 21, 30, 39, 45, 55, 74, 75, 76) s'élargit en direction du trou de logement (2, 3, 22, 23, 29, 36, 37, 43, 44, 54, 71, 72, 73) et qu'
- une douille sphérique (8) destinée à recevoir la vis d'ostéosynthèse (10) est insérée dans le trou de logement (2, 3, 22, 23, 29, 36, 37, 43, 44, 54, 71, 72, 73).

2. Plaque d'ostéosynthèse ou implant orthopédique comparable selon la revendication 1, ***caractérisée en ce que*** le trou de logement (2, 3, 22, 23, 29, 36, 37, 43, 44, 54, 71, 72, 73) et la douille (8) sont sphériques.

3. Plaque d'ostéosynthèse ou implant orthopédique comparable selon la revendication 1 ou 2, ***caractérisée en ce que*** la douille sphérique (8) insérée dans le trou de logement (2, 3, 22, 23, 29, 36, 37, 43, 44, 54, 71, 72, 73) comporte une fente destinée à améliorer la capacité de déformation.

4. Plaque d'ostéosynthèse ou implant orthopédique comparable selon la revendication 1, ***caractérisée en ce qu'***un ou plusieurs évidements (6, 7, 24, 25, 31, 40, 41, 46, 47, 48, 77, 78, 79) sont disposés au sein de la plaque d'ostéosynthèse (1, 20, 28, 35, 53, 70) à la hauteur du trou de logement (2, 3, 22, 23, 29, 36, 37, 43, 44, 54, 71, 72, 73) et du trou de serrage (4, 5, 21, 30, 39, 45, 55, 74, 75, 76) de sorte que le moment d'inertie géométrique de la partie de la plaque d'ostéosynthèse (1, 20, 28, 35, 53, 70) située entre le trou de logement (2, 3, 22, 23, 29, 36, 37, 43, 44, 54, 71, 72, 73) et le trou de serrage (4, 5, 21, 30, 39, 45, 55, 74, 75, 76) est diminué et que, de ce fait, un élargissement du trou de serrage (4, 5, 21, 30, 39, 45, 55, 74, 75, 76) en direction du trou de logement (2, 3, 22, 23, 29, 36, 37, 43, 44, 54, 71, 72, 73) est rendu possible sans appliquer beaucoup de force.

5. Plaque d'ostéosynthèse ou implant orthopédique comparable selon la revendication 1 ou 4, ***caractérisée en ce qu'***un évidement (6, 7, 24, 25, 31, 40, 41, 46, 47, 48, 77, 78, 79) disposé dans la plaque d'ostéosynthèse (1, 20, 28, 35, 53, 70) relie le trou de logement (2, 3, 22, 23, 29, 36, 37, 43, 44, 54, 71, 72, 73) et le trou de serrage (4, 5, 21, 30, 39, 45, 55, 74, 75, 76) associé.

6. Plaque d'ostéosynthèse ou implant orthopédique comparable selon la revendication 1, ***caractérisée en ce qu'***un évidement est conçu de telle sorte dans la plaque d'ostéosynthèse (53), entre un trou de logement (54) et le trou de serrage associé (55), que le trou de logement (54) et le trou de serrage (55) associé sont reliés ensemble et qu'un corps de serrage (56) mobile en coulissement est interposé entre les trous, le corps de serrage (56) faisant partie constitutive des deux parois de trou.

7. Plaque d'ostéosynthèse ou implant orthopédique comparable selon la revendication 6, ***caractérisée en ce que*** le corps de serrage (56) est guidé, mobile en coulissement.
